# EUROPEAN PATENT SPECIFICATION

(11) **EP 2 234 471 B1**
(45) Date of publication and mention of the grant of the patent: **18.02.2015**
(21) Application number: 09701406.2
(22) Date of filing: 07.01.2009
(51) Int. Cl.: A01G 1/00

(54) **METHOD FOR ENRICHING CROPS WITH IODINE, AND CROPS THUS OBTAINED**
VERFAHREN ZUR ANREICHERUNG VON ZUCHTPFLANZEN MIT IODINEN UND DADURCH GEWONNENE ZUCHTPFLANZEN
PROCÉDÉ POUR ENRICHIR DES CULTURES AVEC DE L'IODE, ET CULTURES AINSI OBTENUES

(30) Priority: 09.01.2008 IT BO20080012
(43) Date of publication of application: 06.10.2010
(73) Proprietor: Pizzoli S.P.A., 40054 Budrio (IT)
(72) Inventor: ZANIRATO, Valeria, I-45100 Rovigo (IT); MAYERLE, Marco, I-40032 Camugnano (BO) (IT)
(74) Representative: Zanoli, Enrico
(86) International application number: PCT/EP2009/050142
(87) International publication number: WO 2009/087178

(56) References cited:
- EP-A- 1 153 901
- WO-A-2008/104600
- DATABASE WPI Week 200828 Thomson Scientific, London, GB; AN 2008-D86773 XP002483638 "Planting iodine-containing sweet potato useful as natural source for replenishing iodine to human by putting specified amount of solid iodine organic fertilizer once into center of border check planted with sweet potato seedling" & CN 101 080 986 A (UNIV ZHEJIANG) 5 December 2007 (2007-12-05)
- DATABASE EMBASE [Online] ELSEVIER SCIENCE PUBLISHERS, AMSTERDAM, NL; 1964, ZHURBIN A T: "Addition of iodine to vegetables (russian)" XP002483637 Database accession no. EMB-0008272861 & VOPROSY PITANIIA 1964, vol. 2, 1964, pages 86-87, ISSN: 0042-8833
- HALDIMANN M ET AL: "Iodine content of food groups" JOURNAL OF FOOD COMPOSITION AND ANALYSIS, ACADEMIC PRESS, LONDON, GB, vol. 18, no. 6, 1 September 2005 (2005-09-01), pages 461-471, XP004757200 ISSN: 0889-1575
- Jennifer A. Woolfe: "Sweet Potato; an untapped food resource", 2008, Cambridge University Press page 1,16,24,132, * page 1 * * page 16 * * page 24 * * page 132 *
- Jiu-Lan Dai ET AL: "Selecting iodine-enriched vegetables and the residual effect of iodate application to soil", Biological trace element research, 1 December 2004 (2004-12-01), pages 265-276, XP055130916, United States DOI: 10.1385/BTER:101:3:265 Retrieved from the Internet: URL:http://search.proquest.com/docview/218 365199

## Description

This invention relates to a method for enriching crops with iodine, and disclosed are crops thus obtained. More particularly the invention relates to a method for enriching with iodine crops selected from potatoes, carrots and onions.

Iodine (I) is an important nutrient, as it is an essential component of the thyroid hormones thyroxine (T4) and triiodothyronine (T3), which play a key role in the development of the central nervous system and body accretion.

Under equilibrium conditions, renal excretion is proportional to ingestion (Dunn et al, 1993; Dunn, 1996): the urinary excretion of I (UI) is therefore an accurate indicator of the introduction of I and is the standard method throughout the world to assess I status (WHO, UNICEF & ICCIDD, 1994).

Iodine in foods is primarily in the form of inorganic iodide, which is quickly and almost completely absorbed in the stomach and in the upper part of the small intestine. The I present in foods as iodine is quickly reduced in the intestine and the resulting iodide is promptly absorbed.

Except in rare cases of hypersensitivity to iodide, humans are able to tolerate large quantities of iodine well (Stanbury et al, 1998; Dunn et al, 1998; Institute of Medicine, 2001), thanks to an efficient thyroid control system. Chronic iodine toxicity occurs when the intake of iodide is of 2 mg/day, namely about 15 times greater than the daily requirement, which is of 150 µg/day.

The maximum tolerance level for the introduction of I has been set by the Institute of Medicine of the National Academy of Sciences of the USA at 1100 µg/day for adults (2001). Iodine is a mineral salt widespread in nature but in very reduced percentages.

Environmental iodine deficiency is one of the most serious public health problems, according to estimates by the World Health Organization, as it leads to cretinism, goiter, immune insufficiency and difficulties in learning.

It is estimated that about 2,2 billion people worldwide are suffering from disorders caused by iodine deficiency (International Council for the Control of Iodine Deficiency Disorders, UN estimated 1997). In Italy for example, about 6 million people are exposed to the risks of environmental iodine deficiency, and endemic goiter is present not only in the areas farthest away from the sea, but throughout the nation (Istituto Superiore di Sanità, 1998). The average daily iodine need for an adult is 150 µg/day, and it is higher for pregnant women and children (LARN, SINU).

An adequate nutritional intake of this element, the primary source of which is the diet, is therefore essential. Fish has an especially high content in iodine, but iodine content varies in fruit and vegetables depending on the nature of soil on which it grows, and is often too scarce with respect to human needs.

The average content of iodine in potatoes and carrots is about 2 µg of iodine per 100 grams of edible portion, while the average content of iodine in onions is about 3 µg of iodine per 100 grams of edible onion (Food Standard Agency, London, 2002). From laboratory analyses carried out on samples of potatoes, carrots and onions grown in Italy, however, it emerged that the average content of iodine is less than 1 µg of iodine per 100 grams of virgin crop.

Use of salt fortified with iodine is the most effective strategy for dealing with iodine deficiency disorders, but the consumption of iodized salt is still not widespread.

Another disadvantage of iodized salt is the fact that it cannot be used in sufficient quantities by certain categories of people, such as those with hypertension.

Methods to enrich crops with iodine have been described in several patent and non patent documents.

CN 101080986 (Derwent Abstract 2008-D86773) discloses a method to administer iodine to sweet potatoes by means of a solid fertilizer deposited in holes of the ground. It is also described an alternative method of administration by sprinkling a liquid fertilizer to the leaves of the plants for 5-6 times before sweet potatoes are collected. The amount of fertilizer administered is of 12-150 mg/m², which means no more than 1.5 kg/hectare. Such amount is not referred to iodine but to the fertilizer as such, which means that the amount of iodine administered is substantially less than 1.5 kg/hectare. Also, the need for repeated administration of the fertilizer for 5-6 times is rather disadvantageous.

EP 1 153 901 A2 discloses a composition suitable to increase the content of certain elements or substances in crop, including potatoes, carrots and onions. Elements disclosed are mainly selenium and vanadium, but iodine, zinc and molybdenum are also mentioned, as well as substances such as vitamin C. Aqueous solutions of such elements or substances are sprayed on the leaves of crop to administer desired amounts per hectare of crop. With respect to iodine, aqueous solutions with a iodine content of from 0.000005 to 20 g/l are used. The pH of such solutions is from 5 to 9. Spraying of the solution is repeated more times during the vegetative cycle of crop to administer an amount of iodine of up to 1 kg per hectare. Example 2 teaches to administer a solution of sodium iodide to fruit crop to achieve a total administration of 30 g of iodine per hectare. Such amounts of iodine, however, are not sufficient to achieve a satisfactory enrichment of iodine content in crop such as potatoes, carrots and onions.

WO 2008/104600 A1, an application in the name of the same applicant of the present application and published on 4 September 2008, namely after the priority date of the present application, discloses a method and a composition for enriching potatoes with iodine comprising spraying on potato plants a solution of iodine salts with a iodine content of 0.01 to 50%, preferably 34%, which means from 0.1 to 500 g/l, preferably 340 g/l of iodine. Such solution can be used with 5% phosphorus pentoxide and 12% potassium oxide. The solution is applied on potato crop in such a way to distribute up to 17 kg of iodine per hectare. If a solution with the preferred concentration of 340 g/l is used, the administration of 17 kg of iodine per hectare of soil requires the use of 50 liters of solution (17,000g : 340g/l = 50 l). However, it is difficult to uniformly spray 50 liters of solution on a surface of one hectare (10,000 m²), for the amount of liquid is too small compared to the size of the surface to be treated. Also, the pH of such preferred solution containing 34% of iodine, 5% phosphorus pentoxide and 12% potassium oxide is of about 0, and it may not be desirable to administer to crop a solution that is extremely acid.

Thus, a need is felt to improve known methods for enriching with iodine crop such as potato, carrot and onion. In its broadest sense, the invention concerns subject-matter as defined in the appended claims.

An object of the invention is to disclose an improved method for enriching crop with iodine, more particularly a method that is capable of providing a significant enrichment of crop with iodine without a need for repeated treatment during crop growth.

Another object of the invention is to disclose an improved method for enriching crop with iodine, suitable to provide a desired amount of iodine per unit of surface in a single application.

A further object of the invention is to disclose an improved method for enriching crop with iodine, having a desirable pH and providing also a plant nutrient.

A still further object of the present invention is to provide potatoes, carrots and onions enriched with iodine to obtain functional foods useful for health and nutritional wellness.

With the term "functional food" it is meant any fresh or processed food having a health-promoting and/or disease-preventing property beyond the basic nutritional function of supplying nutrients. In the case of iodine, a functional food is a food that provides at least 15% of the RDA (Recommended Daily Allowance), namely at least 22.5 µg/day of iodine.

The above and other objects and advantages of the invention are achieved with a method for enriching crop with iodine during cultivation on soil, characterized in that:
a) said crop is selected from the group consisting of potatoes, carrots and onions;
b) said method comprises administering an aqueous solution comprising:
   i. a iodine salt with a concentration of iodine from 2 to 45 g/l;
   ii. an amount of phosphoric acid to adjust the pH of said solution in the range from 1.5 to 5;
c) said aqueous solution being applied to crop in a volume such that an amount of from 1.8 to 20 kg of iodine is administered per hectare of soil.

Potato is a tuberous crop from herbaceous potato plant. The edible part is a tuber which can be enriched with iodine by administration of an iodine-containing solution to both leaves and tuber via the ground.

Carrots is a root vegetable in which the edible part is the root, and onion is a bulb vegetable in which the edible part is the bulb. They can be enriched with iodine by administration of an iodine-containing solution to leaves and the root or bulb, to these latter via the ground.

Preferably the aqueous solution of a iodine salt has a concentration of iodine from 2 to 45 g/l, more preferably from 3 to 38 g/l.

Preferably, the method according to the invention comprises applying an aqueous solution comprising a iodine salt so that an amount of from 1.8 to 20 kg of iodine per hectare of soil.

With respect to potato crop, the method preferably comprises using an aqueous solution comprising at least 10 g/l of iodine to administer from 5 to 20 kg of iodine per hectare of soil, even more preferably from 6 to 18 kg. Such amount of iodine can be administered in a single application and ensures an accumulation of iodine in the edible potato tuber of at least 22,5 µg of iodine per 100 g of potato, corresponding to 15 % of the RDA. This means the iodine content of enriched potatoes is of at least 22 times the iodine content of traditional, non-treated potatoes.

With respect to carrot crop, the method preferably comprises using an aqueous solution comprising at least 3 g/l of iodine to administer from 1.5 to 12 kg of iodine per hectare of soil, even more preferably from 1.8 to 10 kg of iodine per hectare of soil. Such amount of iodine can be administered in a single application and ensures an accumulation of iodine in the edible carrot root of at least 22.5 µg of iodine per 100 g of carrot, corresponding to 15% of the RDA. This means the iodine content of enriched carrots is of at least 22 times the iodine content of traditional, non-treated carrots.

With respect to onion crop, the method preferably comprises using an aqueous solution comprising at least 5 g/l of iodine to administer from 2 to 20 kg of iodine per hectare of soil, even more preferably from 3 to 18 kg of iodine per hectare of soil. Such amount of iodine ensures an accumulation of iodine in the edible onion bulb of at least 22.5 µg of iodine per 100 g of onion, corresponding to 15% of the RDA. This means the iodine content of enriched onions is 22 times the iodine content of traditional, non-treated onions.

The maximum amount of iodine per hectare of soil used in the method of the invention is of 20 kg, as disclosed above. By administering such amount of iodine it is possible to enrich the level of iodine with respect to traditional, untreated crop of several orders of magnitude. Enrichment with too high amounts of iodine are not of interest in the preparation of functional food.

The solution of iodine salts includes one or more iodine salts selected from the group consisting of: sodium iodides and iodates, calcium iodides and iodates, magnesium iodides and iodates, ammonium iodides and iodates, zinc iodides and iodates, copper iodides and iodates, barium iodides and iodates, cesium iodides and iodates, iodic anhydride, iodic acid, hydriodic acid, and methyl iodide, but preferably potassium iodide.

Since the aqueous solution of most iodine salts mentioned above, particularly of potassium iodide KI, has a basic pH, it has been found advantageous to modify the pH by using phosphoric acid H₃PO₄. For any concentration of iodine within the range from 2 to 45 g/l, the solution contains an amount of H₃PO₄ sufficient to adjust the pH within the range from 1.5 to 5.

A solution having a composition as defined above contains an optimized amount of iodine for a single administration to a crop field, provides plants with phosphorous, which is a plant nutrient, and has a desired acidity to enhance absorption from plants without being too acidic.

Further, the composition optionally contains one or more substances such as surfactants, chelating agents, wetting agents, penetrating agents, humic acids, fulvic acids, amino acids, and others in any suitable combination and amount.

It has been found that potato, carrot and onion crop enriched with iodine according to the method of the invention substantially maintain the increased iodine content even after cooking and/or after storage, with unaffected organoleptic properties.

The method of administration of the iodine solution is of a usual agronomic type, preferably by foliar application, namely by spreading, sprinkling or spraying the solution onto the plants while they grow on fields. Spraying the solution is preferred since it allows to achieve a more uniform distribution of the solution to crop plants. Administration of the iodine solution by spraying it on the epigeal apparatus of the potato, carrot and onion plants, is carried out preferably during the growing season of the plants. The solution is preferably sprayed in form of droplets of a diameter of less than 1 mm. The water used for the preparation of the solution is distilled water, and/or bidistilled water, and/or drinking water, and/or well water, and/or pulping water, and/or channel water, and/or river water, and others.

The solution can be applied to a field by using a spraying bar provided with a plurality of spraying nozzles for foliar applications. The spraying bar is connected to a tank containing the solution to be applied. Tank and spraying bar can be located aboard a tractor, as usual in agricultural treatments. Uniform spraying is necessary to maximize contact of the solution with leaves and absorption by plants.

The administration method is entirely natural since it uses the natural metabolic pathways of the potato, carrot and onion plants. The iodine-containing solution is absorbed by the potato, carrot or onion plants, and effectively accumulated in the tuber, root or bulb respectively, in quantities significantly above the usual content.

Iodine-enriched potatoes, carrots and onions can be used to prepare functional foods and to industrially transform them to obtain functional products based on iodine-enriched potatoes, carrots and onions. The increased iodine content of potatoes, carrots and onions is retained after cooking and after storage, with unaltered organoleptic properties.

### EXAMPLES

### FOLIAR APPLICATION

### Potato crop

### Example 1

A water solution of potassium iodide with a concentration of iodine of 17 g/l was prepared. Phosphoric acid was added to adjust the pH of the solution within the range 1.5-5. The concentration of phosphorous in the solution was of 2.5 g/l expressed as P₂O₅. The pH of the solution was of about 2.

500 liters of such solution were sprayed on 1 hectare of potato plants. The amount of iodine administered was thus of 17 g/l · 500 l= 8,5 kg I/hectare.

Potato crop was harvested after 1 week from administration and iodine content in tubers was determined. The value found was of 25 µg/100g potato. Virgin, untreated potatoes having a iodine content of 1 µg/100g potato were used as comparison. The concentration of iodine in the enriched potatoes was thus is of 25 times the content of virgin, untreated potatoes. This level of enrichment is sufficient to classify the enriched potatoes as a functional food, since it provides at least 15% of the RDA (Recommended Daily Allowance) of iodine.

### Example 2

The method of Example 1 was repeated but a solution with a concentration of I of 34 g/l was used. The concentration of phosphorous in the solution was of 5 g/l expressed as P₂O₅. The pH of the solution was of about 1.7.

500 liters of such solution were sprayed on 1 hectare of potato plants. The amount of iodine administered was thus of 34 g/l · 500 l = 17 kg I/hectare.

The value of iodine concentration found in crop was of 90 µg/100g potato. Such value is of about 90 times the content of virgin, untreated potatoes. This level of enrichment is sufficient to provide about 2/3 of the RDA.

### Comparative example

The method of Example 1 was repeated but a solution with a concentration of I of 1.36 g/l was used.

500 liters of such solution were sprayed on 1 hectare of potato plants. The amount of iodine administered was thus of 1.36 g/l · 500 l= 0.68 kg I/hectare.

The value of iodine concentration found in crop was of 4 µg/100g potato. Although such value is higher than iodine content of virgin, untreated potatoes, it is not sufficient to obtain iodine enriched potatoes as functional food.

### Carrot crop

### Example 3

A water solution of potassium iodide with a concentration of 5 g/l (I) was prepared. Phosphoric acid was added to adjust the pH of the solution within the range 1.5-5. The concentration of phosphorous in the solution was of 0.73 g/l expressed as P₂O₅. The pH of the solution was of about 4.5.

500 liters of such solution were sprayed on 1 hectare of carrot crop. The amount of iodine administered was thus of 5 g/l · 500 l= 2,5 kg I/hectare.

Carrot crop was harvested after 1 week from administration and iodine content in roots was determined. The value found was of 26.3 µg/100g carrot. Virgin, untreated carrots had a iodine content of less than 1 µg/100g carrot.

### Example 4

The method of Example 3 was repeated but a solution with a concentration of I of 6.8 g/l was used. Phosphoric acid was added to adjust the pH of the solution within the range 1.5-5. The concentration of phosphorous in the solution was of 1 g/l expressed as P₂O₅. The pH of the solution was of about 2.8.

500 liters of such solution were sprayed on 1 hectare of carrot crop. The amount of iodine administered was thus of 6.8 g/l · 500 l= 3,4 kg I/hectare.

The value of iodine concentration found in crop was of 35,7 µg/100g carrot.

### Example 5

The method of Example 3 was repeated but a solution with a concentration of I of 10.2 g/l was used). Phosphoric acid was added to adjust the pH of the solution within the range 1.5-5. The concentration of phosphorous in the solution was of 1.5 g/l expressed as P₂O₅. The pH of the solution was of about 2.3.

500 liters of such solution were sprayed on 1 hectare of carrot crop. The amount of iodine administered was thus of 10.2 g/l · 5001 = 5,1 kg I/hectare.

The value of iodine concentration found in crop was of 82.7 µg/100g carrot, which is a quite high value with respect to the typical iodine content of virgin, untreated carrots.

### Example 6

The method of Example 3 was repeated but a solution with a concentration of I of 17.0 g/l was used Phosphoric acid was added to adjust the pH of the solution within the range 1.5-5. The concentration of phosphorous in the solution was of 2.5 g/l expressed as P₂O₅. The pH of the solution was of about 2.

500 liters of such solution were sprayed on 1 hectare of carrot crop. The amount of iodine administered was thus of 17 g/l · 500 l= 8,5 kg I/hectare.

The value of iodine concentration found in crop was of 226 µg/100g carrot, which is a very high value with respect to the typical iodine content of virgin, untreated carrots.

### Onion crop

### Example 7

A water solution of potassium iodide with a concentration of 28.6 g/l (I) was prepared. Phosphoric acid was added to adjust the pH of the solution within the range 1.5-5. The concentration of phosphorous in the solution was of 4.2 g/l expressed as P₂O₅. The pH of the solution was of about 1.8.

500 liters of such solution were sprayed on 1 hectare of onion crop. The amount of iodine administered was thus of 28.6 g/l · 500 l = 14,3 kg I/hectare.

Onion crop was harvested after 4 days from administration and iodine content in bulbs was determined. The value found was of 44 µg/100g onion. Virgin, untreated onions had a iodine content of less than 1 µg/100 g onions.

### STORAGE AND COOKING

Tests were carried out on iodine enriched crop to determine effect of storage and cooking on iodine content. The results show that there is no significant decrease caused by cooking and storage.

### Example 8

Iodine enriched potatoes with an average iodine content of 46 µg/100g potato were steam cooked for 20 minutes from the time at which water began to boil. Cooked potatoes had a iodine content of about 53 µg/100g potato.

### Example 9

The cooking test of example 8 was repeated using iodine enriched potatoes with an average iodine content of 38 µg/100g potato. Cooked potatoes had a iodine content of about 37 µg/100g potato.

### Example 10

Iodine enriched potatoes with an average iodine content of 39 µg/100g potato were kept for 4 months in a dark room at a controlled temperature of about 6°C. Average iodine content at the end of the 4-month period was of 41 µg/100g potato.

This shows that iodine content does not significantly vary over time.

### Example 11

Iodine enriched carrots with an average iodine content of 73 µg/100g carrot were kept for 2 months in a dark room at a controlled temperature of about 6°C. Average iodine content at the end of the 2-month period was of 91 µg/100g carrot.

This shows that iodine content does not significantly vary over time.

Table 1 below shows a summary of the results of the examples above on foliar application.

**Table 1**

| | Potato | | | Carrot | | | | Onion |
|---|---|---|---|---|---|---|---|---|
| | Ex. 1 | Ex. 2 | C. Ex. | Ex. 3 | Ex. 4 | Ex. 5 | Ex. 6 | Ex. 7 |
| Conc. of solution used as g/l Iodine | **17** | **34** | **1.36** | **5.0** | **6.8** | **10.2** | **17** | **28.6** |
| Kg iodine administered /hectare | **8.5** | **17** | **0.68** | **2.3** | **3.4** | **5.1** | **8.5** | **14.3** |
| Liters of solution sprayed/hectare | **500** | **500** | **500** | **500** | **500** | **500** | **500** | **500** |
| µg/100g of enriched crop | **25** | **90** | **4** | **26.3** | **35.7** | **82.7** | **226** | **44** |
| µg/100g of virgin crop (comparison) | **1** | **1** | **1** | **< 1** | **< 1** | **< 1** | **< 1** | **< 1** |

As it appears from the examples above, the method according to the invention is effective to enrich crop with iodine even by a single foliar application to crop plants during growth on the field. The iodine concentration of the solution can be varied within the claimed range to administer a desired amount of iodine per hectare of soil. The volume of the solution in liters is selected to have a sufficient amount of water to be sprayed on 1 hectare of soil. The experiments have been carried out with 500 liters of solution but such amount can be varied so that the amount of water is sufficient to wet the leaves of the plants and the soil, ensuring optimal contact with iodine and phosphorous source. Also, use of too large volumes of solution is unnecessary, since this would required repeated applications. Use of phosphoric acid provides for pH adjustment and plant nutrition by supply of phosphorous.

## Claims

1. Method for enriching crop with iodine during cultivation on soil, **characterized in that**:
a) said crop is selected from the group consisting of potatoes, carrots and onions;
b) said method comprises administering an aqueous solution comprising:
i) a iodine salt with a concentration of iodine from 2 to 45 g/l;
ii) an amount of phosphoric acid adjusting the pH of such solution in the range from 1.5 to 5;
c) said aqueous solution is applied to crop in a volume such that an amount of from 1.8 to 20 kg of iodine is administered per hectare of soil.

2. Method according to claim 1, **characterized in that** said crop is potato crop, and the method comprises applying an aqueous solution comprising at least 10 g/l of iodine.

3. Method according to claim 2, **characterised by** administering from 5 to 20 kg of iodine per hectare of soil.

4. Method according to claim 2, **characterised by** administering from 6 to 18 kg of iodine per hectare of soil.

5. Method according to claim 2, **characterised by** administering from 6 to 18 kg of iodine per hectare of soil in a single application.

6. Method according to claim 1, **characterized in that** said crop is carrot crop, and the method comprises applying an aqueous solution comprising at least 3 g/l of iodine.

7. Method according to claim 6, **characterised by** administering from 1.5 to 12 kg of iodine per hectare of soil.

8. Method according to claim 6, **characterised by** administering from 1.8 to 10 kg of iodine per hectare of soil.

9. Method according to claim 6, **characterised by** administering from 1.8 to 10 kg of iodine per hectare of soil in a single application.

10. Method according to claim 1, **characterized in that** said crop is onion crop, and the method comprises applying an aqueous solution comprising at least 5 g/l of iodine.

11. Method according to claim 10, **characterised by** administering from 2 to 20 kg of iodine per hectare of soil.

12. Method according to claim 10, **characterised by** administering from 3 to 18 kg of iodine per hectare of soil.

13. Method according to claim 10, **characterised by** administering from 3 kg to 18 kg of iodine per hectare of soil in a single application.

14. Method according to claim 1, **characterized in that** said aqueous solution of a iodine salt has a concentration of iodine from 3 to 38 g/l.

## Patentansprüche

1. Verfahren zum Anreichern von Feldfrüchten mit Iod während der Kultivierung auf einem Boden, **dadurch gekennzeichnet, dass**:
a) die Feldfrüchte aus der Gruppe ausgewählt sind, die aus Kartoffeln, Möhren und Zwiebeln besteht;
b) das Verfahren das Anwenden einer wässrigen Lösung umfasst, die umfasst:
i) ein Iodsalz mit einer Iodkonzentration von 2 bis 45 g/l;
ii) einen Anteil Phosphorsäure, der den pH dieser Lösung auf den Bereich von 1,5 bis 5 einstellt;
c) die Lösung in einem solchen Volumen auf die Feldfrüchte aufgebracht wird, dass eine Menge von 1,8 bis 20 kg Iod pro Hektar Boden aufgebracht wird.

2. Verfahren nach Anspruch 1, **dadurch gekennzeichnet, dass** die Feldfrüchte Kartoffeln sind und das Verfahren das Aufbringen einer wässrigen Lösung umfasst, die mindestens 10 g/l Iod enthält.

3. Verfahren nach Anspruch 1, **dadurch gekennzeichnet, dass** 5 bis 20 kg Iod pro Hektar Boden aufgebracht werden.

4. Verfahren nach Anspruch 2, **dadurch gekennzeichnet, dass** 6 bis 18 kg Iod pro Hektar Boden aufgebracht werden.

5. Verfahren nach Anspruch 2, **dadurch gekennzeichnet, dass** 6 bis 18 kg Iod pro Hektar Boden in einer einzelnen Anwendung aufgebracht werden.

6. Verfahren nach Anspruch 1, **dadurch gekennzeichnet, dass** die Feldfrüchte Möhren sind und das Verfahren das Aufbringen einer wässrigen Lösung umfasst, die mindestens 3 g/l Iod enthält.

7. Verfahren nach Anspruch 6, **dadurch gekennzeichnet, dass** 1,5 bis 12 kg Iod pro Hektar Boden aufgebracht werden.

8. Verfahren nach Anspruch 6, **dadurch gekennzeichnet, dass** 1,8 bis 10 kg Iod pro Hektar Boden aufgebracht werden.

9. Verfahren nach Anspruch 6, **dadurch gekennzeichnet, dass** 1,8 bis 10 kg Iod pro Hektar Boden in einer einzigen Anwendung aufgebracht werden.

10. Verfahren nach Anspruch 1, **dadurch gekennzeichnet, dass** die Feldfrüchte Zwiebeln sind und das Verfahren das Aufbringen einer wässrigen Lösung umfasst, die mindestens 5 g/l Iod umfasst.

11. Verfahren nach Anspruch 10, **dadurch gekennzeichnet, dass** 2 bis 20 kg Iod pro Hektar Boden aufgebracht werden.

12. Verfahren nach Anspruch 10, **dadurch gekennzeichnet, dass** 3 bis 18 kg Iod pro Hektar Boden aufgebracht werden.

13. Verfahren nach Anspruch 10, **dadurch gekennzeichnet, dass** 3 kg bis 18 kg Iod pro Hektar Boden in einer einzigen Anwendung aufgebracht werden.

14. Verfahren nach Anspruch 1, **dadurch gekennzeichnet, dass** die wässrige Lösung eines Iodsalzes eine Iodkonzentration von 3 bis 38 g/l aufweist.

## Revendications

1. Procédé destiné à enrichir une culture avec de l'iode au cours d'une culture sur terre, **caractérisé en ce que** :
a) ladite culture est sélectionnée dans le groupe constitué par des pommes de terre, des carottes et des oignons ;
b) ledit procédé comprend une étape consistant à administrer une solution aqueuse qui comprend :
i) un sel d'iode qui présente une concentration en iode comprise entre 2 g / 1 et 45 g / 1;
ii) une quantité d'acide phosphorique qui règle le pH d'une telle solution dans une plage comprise entre 1,5 et 5 ;
c) ladite solution aqueuse est appliquée à la culture en volume de telle sorte qu'une quantité comprise entre 1,8 kg et 20 kg d'iode soit administrée par hectare de terre.

2. Procédé selon la revendication 1, **caractérisé en ce que** ladite culture est une culture de pommes de terre, et le procédé comprend une étape consistant à appliquer une solution aqueuse qui comprend au moins 10 g / 1 d'iode.

3. Procédé selon la revendication 2, **caractérisé par** une étape consistant à administrer entre 5 kg et 20 kg d'iode par hectare de terre.

4. Procédé selon la revendication 2, **caractérisé par** une étape consistant à administrer entre 6 kg et 18 kg d'iode par hectare de terre.

5. Procédé selon la revendication 2, **caractérisé par** une étape consistant à administrer entre 6 kg et 18 kg d'iode par hectare de terre en une seule application.

6. Procédé selon la revendication 1, **caractérisé en ce que** ladite culture est une culture de carottes, et le procédé comprend une étape consistant à appliquer une solution aqueuse qui comprend au moins 3 g/l d'iode.

7. Procédé selon la revendication 6, **caractérisé par** une étape consistant à administrer entre 1,5 kg et 12 kg d'iode par hectare de terre.

8. Procédé selon la revendication 6, **caractérisé par** une étape consistant à administrer entre 1,8 kg et 10 kg d'iode par hectare de terre.

9. Procédé selon la revendication 6, **caractérisé par** une étape consistant à administrer entre 1,8 kg et 10 kg d'iode par hectare de terre en une seule application.

10. Procédé selon la revendication 1, **caractérisé en ce que** ladite culture est une culture d'oignons, et le procédé comprend une étape consistant à appliquer une solution aqueuse qui comprend au moins 5 g / 1 d'iode.

11. Procédé selon la revendication 10, **caractérisé par** une étape consistant à administrer entre 2 kg et 20 kg d'iode par hectare de terre.

12. Procédé selon la revendication 10, **caractérisé par** une étape consistant à administrer entre 3 kg et 18 kg d'iode par hectare de terre.

13. Procédé selon la revendication 10, **caractérisé par** une étape consistant à administrer entre 3 kg et 18 kg d'iode par hectare de terre en une seule application.

14. Procédé selon la revendication 1, **caractérisé en ce que** ladite solution aqueuse d'un sel d'iode présente une concentration d'iode comprise entre 3 g/l et 38g/l.
